(19) Europäisches Patentamt
European Patent Office
Office européen des brevets

(11) Veröffentlichungsnummer: **0 241 834**
**A2**

(12) # EUROPÄISCHE PATENTANMELDUNG

(21) Anmeldenummer: **87105060.5**

(22) Anmeldetag: **06.04.87**

(51) Int. Cl.⁴: **C07D 311/56**, C07D 405/12, C07D 407/12, A01N 43/32

(30) Priorität: **18.04.86 DE 3613065**

(43) Veröffentlichungstag der Anmeldung: **21.10.87 Patentblatt 87/43**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Müller, Nikolaus, Dr.**
**Knipprather Strasse 98**
**D-4019 Monheim(DE)**
Erfinder: **Kranz, Eckart, Dr.**
**Am Acker 9**
**D-5600 Wuppertal 1(DE)**
Erfinder: **Andrews, Peter, Dr.**
**Gellertweg 2**
**D-5600 Wuppertal 1(DE)**

(54) **Verwendung von 3-Carbamoyl-4-hydroxy-cumarinen zur Bekämpfung von parasitären Helminthen, neue 3-Carbamoyl-4-hydroxy-cumarine und ihre Herstellung.**

(57) Die vorliegende Erfindung betrifft die Verwendung von 3-Carbamoyl-4-hydroxycumarinen der allgemeinen Formel I

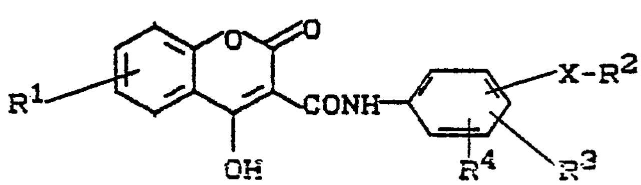

I

in welcher
$R^1$ für Wasserstoff, Halogen, Alkyl, $NO_2$, CN, Alkoxy steht,
X für O, S, SO, $SO_2$ steht
$R^2$ für Alkyl, Halogenalkyl, Phenyl, Naphthyl, Pyridyl die gegebenenfalls substiutiert sein können steht; der Rest -X-$R^2$ kann auch gemein sam mit dem Rest $R^3$ eine Alkylendioxy-(-O-Alk-O), Halogenalkylendioxy-, Oxyalkylen-oxyalkylen-(-O-Alk-O-Alk-), Oxyhalogenalkylen-oxyhalogenalkylen-brücke bilden;
$R^3$ und $R^4$ unabhängig voneinander für Wasserstoff, Halogen, CN, OH, $NO_2$, Amino, Mono-, Dialkylamino Alkyl, Halogenalkyl, Aralkyl, Aryl die gegebenenfalls substituiert sein können stehen
zur Bekämpfung von parasitären Helminthen.

Die vorliegende Erfindung betrifft ferner neue 3-Carbamoyl-4-hydroxycumarine und Verfahren zur ihrer Herstellung.

EP 0 241 834 A2

Xerox Copy Centre

# Verwendung von 3-Carbamoyl-4-hydroxy-cumarinen zur Bekämpfung von parasitären Helminthen, neue 3-Carbamoyl-4-hydroxy-cumarine und ihre Herstellung

Die vorliegende Erfindung betrifft die Verwendung von 3-Carbamoyl-4-hydroxy-cumarinen zur Bekämpfung von parasitären Helminthen, neue 3-Carbamoyl-4-hydroxy-cumarine und ihre Herstellung.

Es sind bereits 3-Carbamoyl-4-hydroxycumarine mit anthelminthischer Wirkung bekannt geworden. Ihre Wirkung befriedigt jedoch vor allem bei niedrigen Aufwandkonzentrationen nicht immer.

1. Es wurde gefunden, daß die 3-Carbamoyl-4-hydroxy-cumarine der allgemeinen Formel

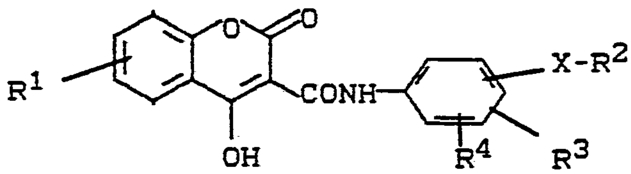

I

in welcher

$R^1$ für Wasserstoff, Halogen, Alkyl, $NO_2$, CN, Alkoxy steht,

X für O, S, SO, $SO_2$ steht

$R^2$ für Alkyl, Halogenalkyl, Phenyl, Naphthyl, Pyridyl die gegebenenfalls substiutiert sein können steht; der Rest -X-$R^2$ kann auch gemeinsam mit dem Rest $R^3$ eine Alkylendioxy-(-O-Alk-O), Halogenalkylendioxy-, Oxyalkylen-oxyalkylen-(-O-Alk-O-Alk-), Oxyhalogenalkylen-oxyhalogenalkylen-brücke bilden;

$R^3$ und $R^4$ unabhängig voneinander für Wasserstoff, Halogen, CN, OH, $NO_2$, Amino, Mono-, Dialkylamino Alkyl, Halogenalkyl, Aralkyl, Aryl die gegebenenfalls substituiert sein können stehen zur Bekämfung von parasitären Helminthen eingesetzt werden können. Die Verbindungen der Formel I können in ihren verschiedenen tautomeren Formen sowie als Gemische dieser tautomeren Formen vorliegen:

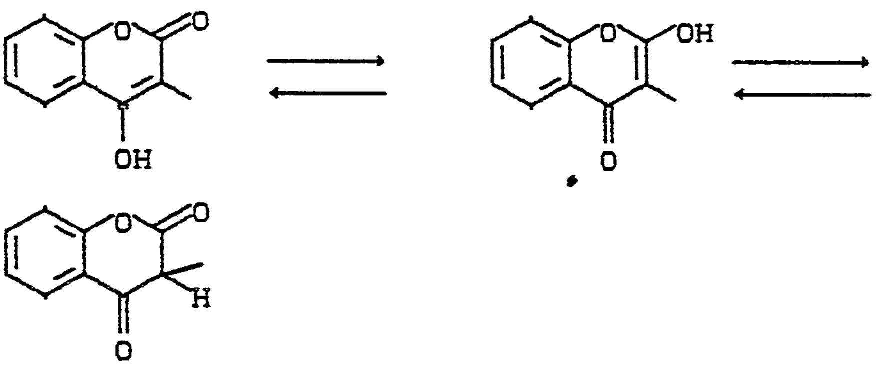

2. Es wurden die neuen 3-Carbamoyl-4-hydroxycumarine der allgemeinen Formel I gefunden

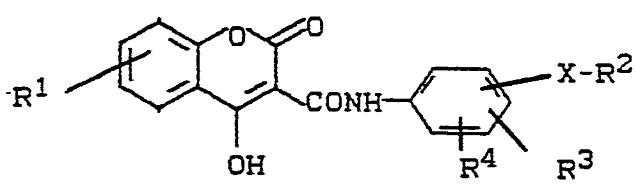

I

in welcher

$R^1$ für Wasserstoff, Halogen, Alkyl, $NO_2$, CN, Alkoxy steht,

X für O, S, SO, $SO_2$ steht,

$R^2$ für Phenyl, Naphthyl, Pyridyl die gegebenenfalls substituiert sein können steht,

$R^3$ und $R^4$ unabhängig voneinander für Wasserstoff, Halogen, CN, OH, $NO_2$, Amino, Mono-, Dialkylamino, Alkyl, Halogenalkyl, Aralkyl, Aryl die gegebenenfalls substituiert sein können stehen,

ausgenommen jedoch 4-Hydroxy-3-[4-(4-chlorphenoxy)-phenyl-carbamoyl]-cumarin.

3. Es wurde gefunden, daß man die 3-Carbamoyl-4-hydroxy-cumarine der Formel I

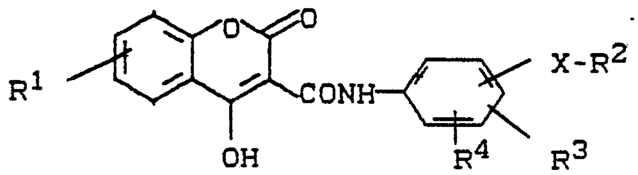

I

in welcher
$R^1$ für Wasserstoff, Halogen, Alkyl, $NO_2$, CN, Alkoxy steht,
X für O, S, SO, $SO_2$ steht,
$R^2$ für Phenyl, Naphthyl, Pyridyl die gegebenenfalls substituiert sein können steht,
$R^3$ und $R^4$ unabhängig voneinander für Wasserstoff, Halogen, CN, OH, $NO_2$, Amino, Mono-, Dialkylamino, Alkyl, Halogenalkyl, Aralkyl, Aryl die gegebenenfalls substituiert sein können stehen,
ausgenommen jedoch 4-Hydroxy-3[4-(4-chlorphenoxy)-phenyl-carbamoyl]-cumarin,
erhält indem man

a) 4-Hydroxycumarine der Formel II

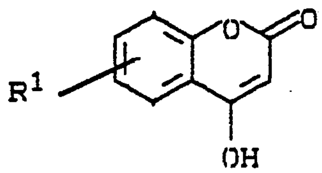

II

in welcher
$R^1$ die oben angegebene Bedeutung hat mit Isocyanaten der Formel III

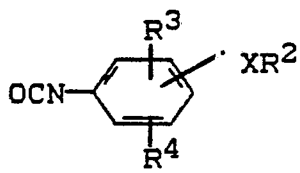

III

in welcher
X, $R^2$, $R^3$, $R^4$ die oben angegebene Bedeutung haben
umsetzt, oder

b) 4-Hydroxycumarin-3-carbonsäureester der Formel IV

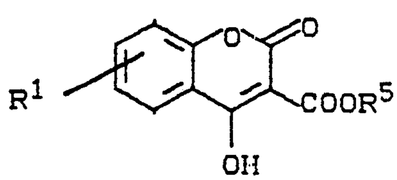

IV

in welcher
$R^1$ die oben angegebene Bedeutung hat,
$R^5$ für $C_{1-4}$-Alkyl, Phenyl, 4-$NO_2$-Phenyl steht mit Aminen der Formel VI

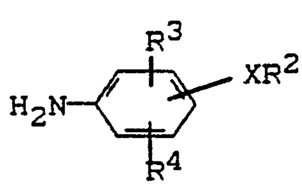

V

in welcher

X, $R^2$, $R^3$ $R^4$ die oben angegebene Bedeutung besitzt umsetzt.

Bevorzugt werden Verbindungen der Formel I eingesetzt in welcher

$R^1$ für Wasserstoff, Fluor, Chlor, Brom, $C_{1-4}$-Alkyl, $NO_2$, CN, $C_{1-4}$-Alkoxy steht,

X für O oder S steht,

$R^2$ für $C_{1-4}$-Alkyl, $C_{1-4}$-Halogenalkyl, Phenyl, Naphthyl, Pyridyl steht, die gegebenenfalls durch einen oder mehrere der folgenden Reste substituiert sind: Alkyl mit vorzugsweise 1 bis 4, insbesondere 1 oder 2 Kohlenstoffatomen, wie Methyl, Ethyl, n.-und i.-Propyl und n.-, i.-, s.-und t.-Butyl; Alkoxy mit vorzugsweise 1 bis 4, insbesondere 1 oder 2 Kohlenstoffatomen, wie Methoxy, Ethoxy, n.-und i.-Propyloxy und n.-, i.-, s.-und t.-Butyloxy; Alkylthio mit vorzugsweise 1 bis 4, insbesondere 1 oder 2 Kohlenstoffatomen, wie Methylthio, Ethylthio, n.-und i.-Propylthio und n.-, i.-, s.-und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Fluorchlorethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen wie Trifluormethoxy; Halogenalkylthio mit vorzugsweise 1 bis 4, insbesonder 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylthio; Alkylendioxy mit vorzugsweise 1 oder 2 Kohlenstoffatomen wie Methylendioxy oder Ethylendioxy; halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugsweise Fluor oder Chlor, insbesondere Fluor stehen wie Difluormethylendioxy, Trifluorethylendioxy, Tetrafluorethylendioxy; Oxyalkylen-oxyalkylen, insbesondere Oxymethylen-oxymethylen ($-O-CH_2-O-CH_2-$) die gegebenenfalls ein oder mehrfach durch Halogen insbesondere Fluor oder Chlor substituiert sein können, vorzugsweise Oxydifluormethylen-oxydifluormethylen; Hydroxy; Halogen, vorzugweise Fluor, Chlor, Brom und Jod, insbesondere Chlor und Brom; Cyano; Nitro; Amino; Monoalkyl-und Dialkylamino mit vorzugsweise 1 bis 4, insbesondere 1 oder 2 Kohlenstoffatomen je Alkylgruppe, wie Methylamino, Methyl-ethyl-amino, n.-und i.-Propylamino und Methyl-n.-Butylamino; Formyl; Carboxyl; Alkylcarbonyl mit vorzugsweise 2-4 Kohlenstoffatomen insbesondere Acetyl; Carbalkoxy mit vorzugsweise 2 bis 4, insbesonder 2 oder 3 Kohlenstoffatomen, wie Carbomethoxy und Carboethoxy; Sulfo($-SO_3H$); Alkylsulfonyl mit vorzugsweise 1 bis 4, insbesondere 1 oder 2 Kohlenstoffatomen, wie Methylsulfonyl und Ethylsulfonyl; Halogenalkylsulfonyl mit vorzugsweise 1-2 Kohlenstoffatomen udn 1-3 Halogen atomen wobei die Halogenatome gleich oder verschieden sind und als Halogenatome vorzugsweise Fluor oder Chlor stehen wie Trichlormethylsulfonyl oder Trifluormethylsulfonyl; Arylsulfonyl mit vorzugsweise 6 oder 10 Arylkohlenstoffatomen, wie Phenylsulfonyl; Phenyl, Napthyl, Phenoxy, Naphthoxy, Phenylthio, Naphthylthio, die ihrerseits wieder substituiert sein können.

Bevorzugt steht der Rest $-X-R^2$ mit einem dazu in ortho Stellung stehenden Rest $R^3$ für eine Alkylendioxy-insbesondere Methylendioxy-oder Ethylendioxy-brücke, oder eine Oxyalkylenoxyalkylen-insbesondere Oxymethylenoxymethylenbrücke, die gegebenenfalls durch ein oder mehrere gleiche oder verschiedene Halogenatome insbesondere Fluor oder Chlor substituiert ist.

Besonders bevorzugt seien folgende Brücken genannt: Methylendioxy, Ethylendioxy, Difluormethylendioxy, Dichlormethylendioxy, Tetrafluorethylendioxy, Trifluorchlorethylendioxy, Oxymethylenoxymethylen, Oxydifluormethylenoxydifluormethylen.

$R^3$ und $R^4$ stehen unabhängig voneinader bevorzugt für

Wasserstoff, Halogen insbesondere Fluor, Chlor, Brom, CN, OH, $NO_2$, Amino, Monoalkyl-und Dialkylamino mit vorzugsweise 1 bis 4, insbesondere 1 oder 2 Kohlenstoffatomen je Alkylgruppe, wie Methylamino, Methyl-ethyl-amino, n.-und i.-Propylamino und Methyl-n.-Butylamino; Alkyl mit vorzugsweise 1 bis 4, insbesondere 1 oder 2 Kohlenstoffatomen, wie Methyl, Ethyl, n.-und i.-Propyl und n.-i.-, s.-und t.-Butyl; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Fluorchlorethyl; Aralkyl mit 1-4 Kohlenstoffatomen insbesonder 1-2 Kohlenstoffatomen im Alkylteil und bevorzugt Phenyl im Arylteil, das bevorzugt durch eine der weiter oben angegebenen Substituenten substituiert sein kann, Phenyl, Naphthyl, Pyridyl, die bevorzugt durch einen der weiter oben angegebenen Substituenten substiutiert sein können.

Besonders bevorzugt werden Verbindungen der Formel I eingesetzt,
in welcher
$R^1$ für Wasserstoff oder Methyl steht,
X für O oder S steht,
$R^2$ für $C_{1-4}$-Alkyl insbesondere Methyl, $C_{1-4}$ -Halogenalkyl insbesondere Trifluormethyl, steht, für Phenyl oder Pyridyl steht die gegebenenfalls durch einen oder mehrere der folgenden Reste substituiert sind:
$C_1.C_4$-Alkyl, insbesondere Methyl, $C_1.C_4$-Alkoxy, insbesondere Methoxy, Ethoxy, $C_1.C_4$-Halogenalkoxy, insbesondere Trifluormethoxy, Fluor-chlorethoxy, $C_1.C_4$-Halogenalkylthio, insbesondere Trifluormethylthio, $C_1.C_4$-Alkylthio, insbesondere Methylthio, $C_1.C_4$-Alkylsulfonyl, insbesondere Methylsulfonyl, $C_1.C_4$-Halogenalkylsulfonyl, insbesondere Trifluormethylsulfonyl, $C_1.C_4$-Halogenalkyl, insbesondere Trifluormethyl, Methylendioxy oder Ethylendioxy, die gegebenenfalls durch Fluor oder Chlor substituiert sind, Halogen, insbesondere Fluor oder Chlor, $NO_2$.

$R^3$ und $R^4$ unabhängig voneinander für Wasserstoff, Halogen, insbesondere Chlor oder Fluor, $C_{1-4}$-Alkyl insbesondere Methyl, $C_{1-4}$-Halogenalkyl insbesondere Trifluormethyl stehen.

Ganz besonders bevorzugt werden Verbindungen der Formel I eingesetzt
in welcher
$R^1$ für Wasserstoff oder Methyl steht,
X für O steht,
$R^2$ für Phenyl steht, das gegebenenfalls durch einen oder mehrere der obengenannten Reste insbesondere durch $C_{1-4}$-Halogenalkyl insbesondere Trifluormethyl substiutiert ist,
$R^3$ und $R^4$ für Wasserstoff stehen.

Die neuen 3-Carbamoyl-4-hydroxycumarine gemäß 2 (oben) werden erhalten indem man z.B. gemäß Verfahren 3a) 4-Hydoxycumarin mit Trifluormethylphenoxyphenylisocyanat umsetzt.

Der Reaktionsablauf kann durch folgendes Formelschema wiedergegeben werden:

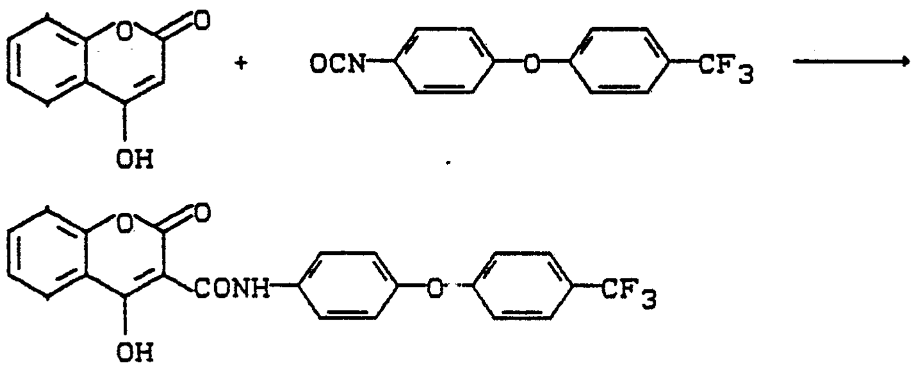

Die für das erfindungsgemäße Verfahren als Ausgangsstoffe benötigten Isocyanate sind durch die Formel (III) allgemein defniert. In dieser Formel stehen X, $R^2$, $R^3$, $R^4$ vorzugsweise für die Reste, die bei den Stoffen der Formel (I) bereits für diese substituenten vorzugsweise genannt wurden.

Im einzelnen seien folgende Isocyanate der Formel III genannt:
4-Trifluormethoxyphenylisocyanat, 4-Trifluormethylthiophenylisocyanat, 3-Chlor-4-trifluormethylphenylisocyanat, 4-Trifluormethylsulfonylphenylisocyanat,
4-Trifluorethoxyphenylisocyanat, 4-Methoxy-, 4-Trifluormethoxy-und 4-tri fluormethylthiophenoxyphenylisocyanat, 4-Trifluormethylphenoxy-phenylisocyanat.

Die Isocyanate der Formel (III) sind bekannt, bzw. lassen sie sich in allgemein bekannter Art und Weise herstellen.

Die außerdem als Ausgangsstoffe zu verwendenden 4-Hydroxycumarine der Formel II sind ebenfalls allgemein bekannte Verbindungen der organischen Chemie.

Verbindungen der Formeln II und III werden in Gegenwert von Verdünnungsmitteln und in Gegenwart von Basen sowie gegebenenfalls in Gegenwart weiterer Katalysatoren umgesetzt.

Als Basen seien genannt: Alkali-, Erdalkalialkoholate und tertiäre Amine. Besonders bevorzugt seien folgende Basen genannt; Triethylamin, Pyridin, Picoline, Trimethylamin, N-Methylmorpholin, N-Ethylpyrrolidin, Diazabicyclo(4,3,0)-undecen (DBU), 1,4-Diaza-bicyclo-2,2,2-octan (DABCO), Diazabicyclo(3,2,0)nonen (DBN).

als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie 4-Tetrafluorethoxyphenylisocyanat, 4-Methoxy-, 4-Trifluormethoxy-und 4-Tri fluormethylthiophenoxyphenylisocyanat, 4-Trifluormethylphenoxy-phenylisocyanat.

Die Isocyanate der Formel (III) sind bekannt, bzw. lassen sie sich in allgemein bekannter Art und Weise herstellen.

Die außerdem als Ausgangsstoffe zu verwendenden 4-Hydroxycumarine der Formel II sind ebenfalls allgemein bekannte Verbindungen der organischen Chemie.

Verbindungen der Formeln II und III werden in Gegenwart von Verdünnungsmitteln und in Gegenwart von Basen sowie gegebenenfalls in Gegenwart weiterer Katalysatoren umgesetzt.

Als Basen seien genannt: Alkali-, Erdalkalialkoholate und tertiäre Amine. Besoners bevorzugt seien folgende Basen genannt: Triethylamin, Pyridin, Picoline, Trimethylamin, N-Methylmorphin, N-Ethylpyrrolidin, Diazabicyclo(4,3,0)undecen (DBU), 1,4-Diaza-bicyclo-2,2,2-octan (DABCO) Diazabicyclo(3,2,0)nonen (DBN).

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran, und Dioxan, weiterhin Ketone, wie Aceton, Methylethyl-, Methylisopropyl-und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Katalysatoren kommen die bei Umsetzungen mit Isocyanaten üblichen Katalysatoren infrage. Als solche seien genannt: Metallkatalysatoren des Zn, Sn, Pb wie Dibutylzinndilaurat, Dibtylzinndioxid, Zinnoctoat, Bleioctoat, Zinkoctoat, Zinkchlorid, Zinkacetat.

Die Reaktion wird zwischen 0 und 150° C bevorzugt zwischen 20-50°C durchgeführt. Man arbeitet vorzugsweise unter Normaldruck.

Die Verbindungen der Formeln II und III werden in äquimolaren Mengen eingesetzt ein geringer Überschuß der einen oder anderen Komponente bringt keine wesentlichen Vorteile.

Die Aufarbeitung erfolgt in an sich bekannter Weise, z.B. durch Versetzen der Reaktionsmischung mit verdünnter Säure, Abfiltrieren des Produkts oder Abtrennen der organischen Phase und Abdestillieren des Lösungsmittels.

Setzt man bei Verfahren 3 d als Verbindung der Formel IV 4-Hydroxycumarin-3-carbonsäuremethylester und als Amin der Formel V 3-Phenoxy-4-chloranilin ein, so läßt sich das Verfahren durch folgendes Formelschema darstellen:

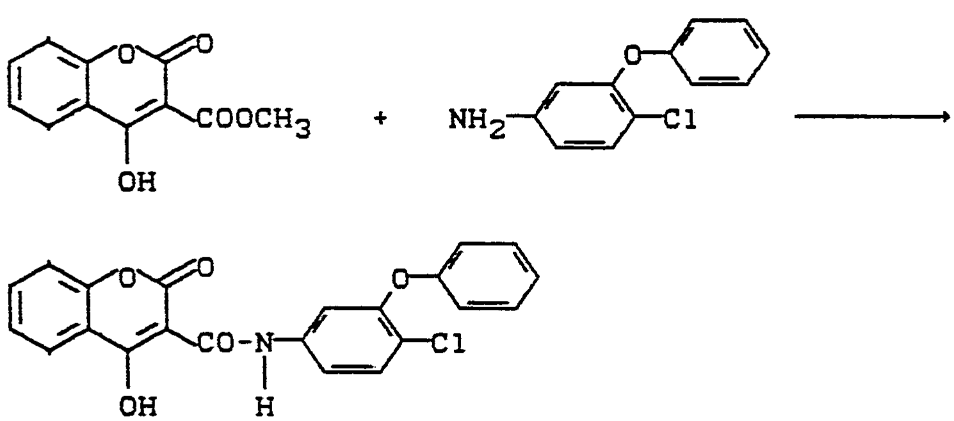

Die Verbindungen der Formel IV sind bekannt oder lassen sich analog zu bekannten Verfahren herstellen. Bevorzugt sind die Verbindungen der Formel IV, in denen $R^1$ die bei den Verbindungen der Formel I als bevorzugt und besonders bevorzugt genannten Bedeutungen besitzt, und bei denen $R^5$ für Methyl, Ethyl, p-Nitrophenyl steht.

Die Amine der Formel V sind bekannt oder lassen sich analog zu bekannten Verfahren herstellen.

Bevorzugt werden Amine der Formel V eingesetzt, in denen die Substituenten X, $R^2$, $R^3$ und $R^4$ die bei den Verbindungen der Formel I genannten bevorzugten und besonders bevorzugten Bedeutungen besitzen.

Im einzelnen seien folgende Verbindungen der Formel V genannt:

4-Trifluormethoxy anilin, 4-Trifluormethylmercaptoanilin, 3-Chlor-4-trifluormethoxy anilin, 3-Chlor-4-trifluormethylmercaptoanilin, 3-Nitro-4-trifluormethoxyanilin, 4-(1,1,2,2-Tetrafluorethoxy)-anilin, 2,6-Dichlor-4-trifluormethylmercapto-anilin, 4-Amino-4'-trifluormethyl-diphenylether, 4-Amino-3'trifluormethyl-diphenylether;

Die Umsetzung der Verbindungen der Formel IV und V erfolgt vorzugsweise in Gegenwart von Verdünnungsmitteln sowie in Gegenwart von Basen.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisbotylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid, ferner Alkohole wie Methanol, Ethanol, Propanol, Butanol.

Als Basen seien genannt Alkali-und Erdalkalihydroxide, Alkali-und Erdalkalialkoholate, insbesondere Natriummethylat oder -ethylat.

Die Reaktion wird zwischen 50 und 150° C bevorzugt zwischen 60-110° C durchgeführt. Man arbeitet vorzugsweise unter Normaldruck.

Die Verbindungen der Formeln VI und VII werden in äquimolaren Mengen eingesetzt ein geringer Überschuß der einen oder anderen Komponente bringt keine wesentlichen Vorteile.

Die Aufarbeitung erfolgt in an sich bekannter Weise, z.B. durch Versetzen der Reaktionsmischung mit Wasser, Ab-trennen der organischen Phase und Abdestillieren des Lösungsmittels.

Wie bereits erwähnt sind die Wirkstoffe der Formel I breit wirksam gegen parasitierende Helminthen. Sie wirken vor allem gegen Trematoden und Nematoden, insbesondere Leberegel und Magen-und Darmnematoden der Wiederkäuer. Darüber hinaus wirken sie auch gegen solche Magen-und Darmnematoden, die gegen die gebräuchlichen Benzimidazol-Anthelmintika resistent und damit nicht mehr ausreichend therapierbar sind.

Die Wirkung wurde im Tierversuch nach oraler, parenteraler und dermaler Applikation bei stark mit Parasiten befallenen Versuchstieren geprüft. Die angewendeten Dosierungen wurden sehr gut von den Versuchstieren vertragen.

Die erfindungsgemäßen Wirkstoffe können als Anthelmintika verwendet werden.

Die erfindungsgemäßen Wirkstoffe können zusammen mit anderen üblichen Anthelmintika verabreicht werden.

Die erfindungsgemäßen Wirkstoffe können entweder als solche oder aber in Kombination mit pharmazeutisch annehmbaren Trägern zur Anwendung gelangen. Als Darreichungsformen in Kombination mit verschiedenen inerten Trägern kommen Tabletten, Kapseln, Granulate, wäßrige Suspensionen, injizierbare Lösungen, Emulsionen und Suspensionen, Elixiere, Sirup, Pasten und dergleichen in Betracht. Derartige Träger umfassen feste Verdünnungsmittel oder Füllstoffe, ein steriles, wäßriges Medium sowie verschiedene nicht toxische organische Lösungsmittel und dergleichen. Selbstverständlich können für eine orale Verabreichung in Betracht kommenden Tabletten und dergleichen mit Süßstoffzusatz und ähnlichen versehen werden. Die therapeutisch wirksame Verbindung soll im vorgenannten Fall in einer Konzentration von etwa 0,5 bis 90 Gew.-% der Gesamtmischung vorhanden sein, d.h. in Mengen, die ausreichend sind, um dem obengenannten Dosierungsspielraum zu erreichen.

Die Formulierungen werden in bekannter Weise hergestellt, z.B. durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nicht toxische organische Lösungsmittel, wie Paraffine (z.B. Erdölfraktionen), pflanzliche Öle (z.B. Erdnuß-(Sesamöl), Alkohole (z.B. Ethylalkohol, Glycerin), Glykole (z.B. Propylenglykol, Polyethylenglykol) und Wasser; feste Trägerstoffe, wie z.B. natürliche Gesteinsmehle (z.B. Kaoline, Tonerden, Talkum, Kreide) synthetische Gesteinsmehle (z.B. hochdisperse Kieselsäure, Silikate), Zucker (z.B. Rohr-, Milch-und

Traubenzucker); Emulgiermittel, wie nicht ionogene und anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Methylcellulose, Stärke und Polyvinylpyrrolidon) und Gleitmittel (z.B. Magnesiumstearat, Talkum, Stearinsäure und Natriumlaurylsulfat).

Im Falle der oralen Anwendung können Tabletten selbstverständlich außer den genannten Trägerstoffen auch Zusätze wie Natriumcitrat, Calciumcarbonat und Dicalciumphosphat, zusammen mit verschidenen Zuschlagstoffen, wie Stärke, vorzugsweise Kartoffelstärke, Gelatine und dergleichen, enthalten. Weiterhin können Gleitmittel, wie Magnesiumstearat, Natriumlaurylsulfat und Talkum, zum Tablettieren mitverwendet werden.

Im Falle wäßriger Suspension und/oder Elixieren, die für die orale Anwendung gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösung Dragees, Ampullen usw. auch in Form von Dosierungseinheiten sein, wobei jede Dosierungseinheit so angepaßt ist, daß sie eine einzelne Dosis des aktiven Bestandteils liefert.

Die erfindungsgemäßen Wirkstoffe können in den Formulierungen auch in Mischungen mit anderen in der Veterinärund/oder Humanmedizin zur Behandlung von Infektionen und/oder Erkrankungen benutzten bekannten Wirkstoffen vorliegen, insbesondere sei genannt L-2,3,5,6-Tetrahydro-6-phenyl-imidazothiazol, Benzimidazolcarbamaten, Praziquantel, Febantel.

Die Wirkstoffe können in üblicher Weise angewendet werden. Die Applikation erfolgt vorzugsweise oral, eine parenterale, insbesondere subkutane, aber auch eine dermale Applikationen (pour-on, spot-on) sind möglich.

Im allgemeinen hat es sich als vorteilhaft erwiesen, Mengen von etwa 1 bis etwa 100 mg der Wirkstoffe je kg Körpergewicht pro Tag zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Inter vall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen. Für die Applikation in der Veterinärmedizin ist der gleiche Dosierungsspielraum vorgesehen. Sinngemäß gelten auch die weiteren obigen Ausführungen.

<u>Beispiel A</u>

In vitro Nematodentest

Caenorhabditis elegans

$10^{-4}$ g Wirkstoff werden in 1 ml Wasser oder 0,1 ml Dimethylsulfoxid (MDSO) gelöst. Diese Lösung wurde auf eine Replica-Platte gegeben. Dazu wurden 2 ml einer E.coli-Suspension gegeben, zu der man 10-20 weibliche Tiere oder Larven von Caenorhabditis elegans in 0,5 ml sterile M9 Pufferlösung gegeben hat. Die E.coli-Suspension wurde hergestellt indem man 300 ml einer Übernachtkultur eines Uracil-bedürftigen E.coli-Stammes mit 1,8 1 steriler M9 Pufferlösung versetzte.

Der Versuchsansatz wurde 7 Tage bei 22° C inkubiert und danach ausgewertet. Es wurde bewertet inwieweit der Wirkstoff die Vermehrung beeinträchtigt und die Konzentration angegeben bei der die Vermehrung verhindert wird. Dabei wurden folgende Ergebnisse erhalten:

Tabelle a

In-vitro Nematodentest

Caenorhabditis elegans

| Wirkstoff Beispiel Nr. | effektive Dosis (µg/ml) |
|---|---|
| 1 | 100 |
| 2 | 100 |
| 3 | 100 |
| 5 | 100 |
| 6 | 100 |
| 7 | 100 |
| 8 | 100 |
| 11 | 100 |

Beispiel B

In-vivro Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen aus der nachfolgenden Tabelle ersichtlich:

Tabelle b)

In-vivo Nematodentest

Haemonchus contortus / Schaf

| Wirkstoff Beispiel Nr. | effektive Dosis in mg/kg |
|---|---|
| 15 | 2,5 |
| 16 | 2,5 |

Herstellungsbeispiele

a) Allgemeine Vorschrift zur Herstellung der Verbindungen der Formel I gemäß Verfahren 3a.
Je 0,03 Mol der Verbindung II und des Isocyanats (III) werden in 120 ml trockenem THF vorgelegt und bei Raumtemperatur innerhalb von 10 Minuten mit einer Lösung von 0,033 Mol "DBU" in 30 ml THF versetzt. Dabei tritt eine leichte Wärmeentwicklung auf. Anschließend wird bis zum vollständigen Umsatz (ca. 4-5 h) am Rückfluß nachgerührt, abgekühlt und der gesamte Ansatz in 400 ml 10 %ige Salzsäure eingerührt. Der dabei sich abscheidende Feststoff wird abgesaugt, mit Wasser gewaschen und getrocknet.

b) Allgemeine Vorschrift zur Herstellung der Verbindungen der Formel I gemäß Verfahren 3d.
0,03 Mol des Ethylesters der Formel IV und 0,03 Mol des Amins der Formel V werden in 100 ml Toluol suspendiert und unter Stickstoff ca. 10 Stunden auf Rückflußtemperatur erhitzt, wobei Ethanol entweicht. Das Reaktionsgemisch wird abgekühlt und der Feststoff abfiltriert. Das abfiltrierte Produkt wird mit Ethanol nachgewaschen und anschließend getrocknet.

Beispiel 1

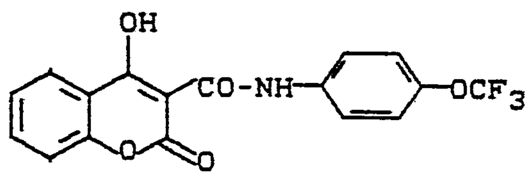

Zu 8,1 g (0,05 Mol) 4-Hydroxycumarin in 100 ml Dimethylsulfoxid werden bei Raumtemperatur zunächst 5,1 g (0,05 Mol) Triethylamin und danach langsam 10,2 g (0,05 Mol) 4-Trifluormethoxyphenylisocyanat zugetropft. Dabei steigt die Temperatur auf 31° C. Man läßt 5 Stunden bei Raumtemperatur nachrühren. Das Reaktionsgemisch wird zu einer Mischung aus 250 ml kaltem Wasser und 15 ml konzentrierter Salzsäure gegeben, wobei ein weißer Niederschlag ausfällt. Man verrührt 30 Minuten unter Kühlung und saugt den Niederschlag ab, wäscht ihn mit saurem Wasser nach und trocknet bei 50° C im Vakuum. Nach Umkristallisation aus 100 ml Dioxan erhält man 8,3 g (45,6 % der Theorie) 4-Hydroxy-3-(4-trifluormethyloxyphenyl-carbamoyl)-cumarin vom Schmelzpunkt 194-195° C.

In analoger Weise können die folgenden Verbindungen der allgemeinen Formel

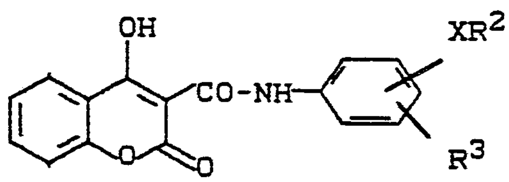

(I)

erhalten werden:

| Bsp. Nr. | $XR^2$ | $R^3$ | Schmelzpunkt (°C) |
|---|---|---|---|
| 2 | $4\text{-}SCF_3$ | 3-Cl | 201-03 |
| 3 | $4\text{-}SCF_2Cl$ | 3-Cl | 206-07 |
| 4 | $4\text{-}OCF_2\text{-}CHFCl$ | - | 210-11 |
| 5 | $4\text{-}SCF_3$ | - | 234-38 |
| 6 | $3,4\text{-}CF_2\text{-}O\text{-}CF_2\text{-}O$ | | 236-42 |
| 7 | $4\text{-}OCF_2\text{-}CHF\text{-}CF_3$ | - | 174-78 |
| 8 | $4\text{-}OCF_2Cl$ | - | 189-92 |
| 9 | $4\text{-}OCH_3$ | $3\text{-}CF_3$ | 285-94 |
| 10 | $4\text{-}OCF_3$ | 2-Cl | 182-92 |
| 11 | $3\text{-}OCF_3$ | - | 160-65 |
| 12 | $4\text{-}OCF_3$ | 3-Cl | 200-04 |
| 13 | 4-O 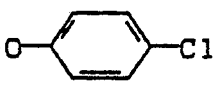 | $3\text{-}CF_3$ | 181-88 |
| 14 | $3,4\text{-}O\text{-}CF_2\text{-}CF_2\text{-}O$ | | 258-63 |
| 15 | 4-O 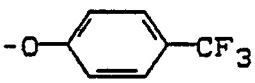 | - | 209-211 |
| 16 | 4-O 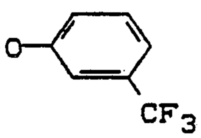 | - | 165 |

**Ansprüche**

1. Verwendung von 3-Carbamoyl-4-hydroxycumarin der allgemeinen Formel I

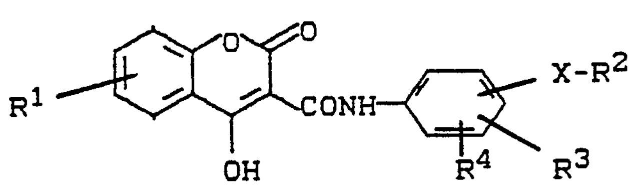

I

in welcher
$R^1$ für Wasserstoff, Halogen, Alkyl, $NO_2$, CN, Alkoxy steht,
X für O, S, SO, $SO_2$ steht
$R^2$ für Alkyl, Halogenalkyl, Phenyl, Naphthyl, Pyridyl die gegebenenfalls substiutiert sein können steht; der Rest $-X-R^2$ kann auch gemeinsam mit dem Rest $R^3$ eine Alkylendioxy-(-O-Alk-O), Halogenalkylendioxy-, Oxyalkylen-oxyalkylen-(-O-Alk-O-Alk-), Oxyhalogenalkylen-oxyhalogenalkylen-brücke bilden;

$R^3$ und $R^4$ unabhängig voneinander für Wasserstoff, Halogen, CN, OH, $NO_2$, Amino, Mono-, Dialkylamino Alkyl, Halogenalkyl, Aralkyl, Aryl die gegebenenfalls substituiert sein können stehen zur Bekämpfung von parasitären Helminthen.

2. 3-Carbamoyl-4-hydroxycumarine der allgemeinen Formel I

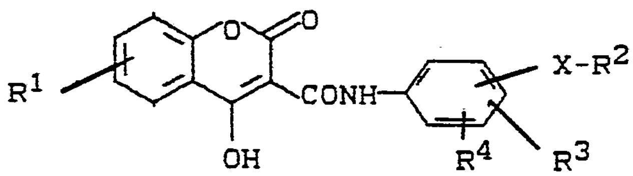

I

in welcher
$R^1$ für Wasserstoff, Halogen, Alkyl, $NO_2$, CN, Alkoxy steht,
X für O, S, SO, $SO_2$ steht,
$R^2$ für Phenyl, Naphthyl, Pyridyl die gegebenenfalls substituiert sein können steht,
$R^3$ und $R^4$ unabhängig voneinander für Wasserstoff, Halogen, CN, OH, $NO_2$, Amino, Mono-, Dialkylamino, Alkyl, Halogenalkyl, Aralkyl, Aryl die gegebenenfalls substituiert sein können stehen,
ausgenommen jedoch 4-Hydroxy-3-[4-(4-chlorphenoxy)-phenyl-carbamoyl]-cumarin.

3. Verfahren zur Herstellung der 3-Carbamoyl-4-hydroxycumarine der Formel I

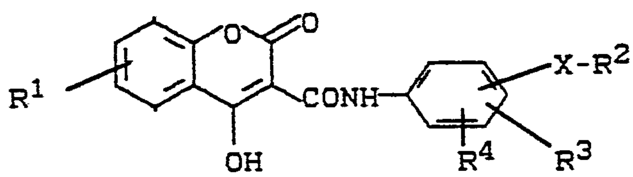

I

in welcher
$R^1$ für Wasserstoff, Halogen, Alkyl, $NO_2$, CN, Alkoxy steht,
X für O, S, SO, $SO_2$ steht,
$R^2$ für Phenyl, Naphthyl, Pyridyl die gegebenenfalls substituiert sein können steht,
$R^3$ und $R^4$ unabhängig voneinander für Wasserstoff, Halogen, CN, OH, $NO_2$, Amino, Mono-, Dialkylamino, Alkyl, Halogenalkyl, Aralkyl, Aryl die gegebenenfalls substituiert sein können stehen,
dadurch gekennzeichnet, daß man

a) 4-Hydroxycumarine der Formel I

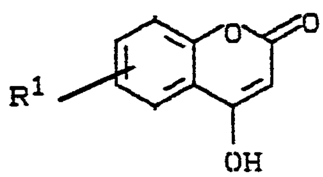

II

in welcher
$R^1$ die oben angegebene Bedeutung hat
mit Isocyanaten der Formel III

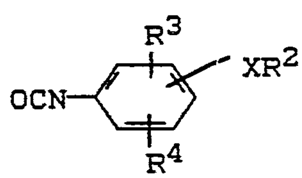

III

in welcher
X, $R^2$, $R^3$, $R^4$ die oben angegebene Bedeutung haben
umsetzt, oder

b) 4-Hydroxycumarin-3-carbonsäureester der Formel IV

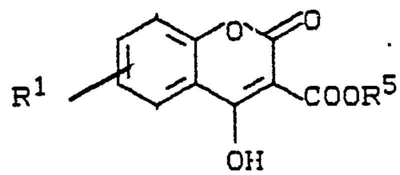

IV

in welcher
$R^1$ die oben angegebene Bedeutung hat,
$R^5$ für $C_{1-4}$-Alkyl, Phenyl, 4-$NO_2$-Phenyl steht mit Aminen der Formel VI

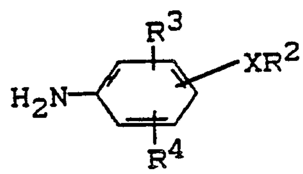

V

in welcher
X, $R^2$, $R^3$ $R^4$ die oben angegebene Bedeutung besitzen
umsetzt.

4. Mittel zur Bekämpfung von parasitären Helminthen, gekennzeichnet durch einen Gehalt an 3-Carbamoyl-4-hydroxycumarinen der Formel I gemäß Anspruch 1.

5. Verfahren zur Herstellung von Mitteln zur Bekämpfung von parasitären Helminthen, dadurch gekennzeichnet, daß man 3-Carbamoyl-4-hydroxycumarine der Formel I gemäß Anspruch 1 mit Lösungsmitteln und/oder Trägerstoffen vermischt.